(19) Europäisches Patentamt
European Patent Office
Office européen des brevets

(11) EP 3 277 259 B1

(12) **EUROPÄISCHE PATENTSCHRIFT**

(45) Veröffentlichungstag und Bekanntmachung des Hinweises auf die Patenterteilung:
**31.07.2019 Patentblatt 2019/31**

(21) Anmeldenummer: **16701526.2**

(22) Anmeldetag: **26.01.2016**

(51) Int Cl.:
***A61K 8/81*** (2006.01) ***A61K 8/41*** (2006.01)
***A61Q 5/04*** (2006.01) ***A61Q 5/06*** (2006.01)

(86) Internationale Anmeldenummer:
**PCT/EP2016/051568**

(87) Internationale Veröffentlichungsnummer:
**WO 2016/155901 (06.10.2016 Gazette 2016/40)**

(54) **MITTEL ENTHALTEND EIN VINYLPYRROLIDON COPOLYMER UND EIN ACRYLAT COPOLYMER**

COMPOSITION CONTAINING A VINYL- AND AN ACRYLATES COPOLYMER

COMPOSITION CONTENANT UN COPOLYMÈRE DE VINYL- ET UNE COPOLYMÈRE D'ACRYLATE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.03.2015 DE 102015205759**

(43) Veröffentlichungstag der Anmeldung:
**07.02.2018 Patentblatt 2018/06**

(73) Patentinhaber: **Henkel AG & Co. KGaA**
**40589 Düsseldorf (DE)**

(72) Erfinder:
- **LANGE, Julia Bibiane**
**24576 Bad Bramstedt (DE)**
- **PULS, Anna**
**21423 Winsen (Luhe) (DE)**
- **MARTINEZ, Cyrielle**
**22765 Hamburg (DE)**
- **RICHTERS, Bernd**
**21129 Hamburg (DE)**

(56) Entgegenhaltungen:
**EP-A2- 1 502 577**
**WO-A2-2010/020503**
**WO-A2-2013/092062**
**DE-A1-102008 059 480**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 277 259 B1

## Beschreibung

Die Anmeldung betrifft das technische Fachgebiet der temporären Umformung keratinhaltiger Fasern, insbesondere menschlicher Haare. Gegenstand der Anmeldung sind kosmetische Mittel, enthaltend mindestens ein Copolymer A auf Basis von Vinylpyrrolidon und mindestens ein Copolymer B auf Basis von Acrylaten. Darüber hinaus sind Gegenstand der vorliegenden Anmeldung die Verwendung dieser kosmetischen Mittel sowie entsprechende Anwendungsverfahren.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, welche sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, welche einer permanenten oder temporären Formgebung der Haare dienen, eine wichtige Rolle. Während bei der permanenten Umformung die chemische Struktur der keratinhaltigen Faser durch Reduktion und Oxidation modifiziert wird, finden solche Modifikationen der chemischen Struktur bei der temporären Umformung nicht statt. Entsprechende Mittel zur temporären Verformung enthalten als festigenden Wirkstoff üblicherweise synthetische Polymere und/oder Wachse.

Die wichtigste Eigenschaft eines Mittels zur temporären Verformung keratinischer Fasern, im Folgenden auch Stylingmittel genannt, besteht darin, den behandelten Fasern in der umgeformten Form - d.h. einer den Fasern aufgeprägten Form - einen möglichst starken Halt zu geben. Handelt es sich bei den keratinischen Fasern um menschliche Haare, spricht man auch von starkem Frisurenhalt oder von hohen Haltegrad des Stylingmittels. Der Frisurenhalt wird im Wesentlichen durch die Art und Menge der eingesetzten festigenden Wirkstoffe bestimmt, wobei jedoch auch ein Einfluss der weiteren Bestandteile des Stylingmittels sowie der Applikationsform gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. des versprühten Aerosols oder Non-Aerosols, und Eigenschaften, welche die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurde im Stand der Technik eine Vielzahl von synthetischen Polymeren als festigende Wirkstoffe entwickelt, welche in Stylingmitteln zur Anwendung kommen. Siehe z.B. WO2010/020503 und WO2013/092062.

Die Polymere lassen sich in kationische, anionische, nichtionische und amphotere festigende Polymere unterteilen. Idealerweise ergeben die Polymere bei der Anwendung auf dem Haar einen Polymerfilm, welcher einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken bzw. Rückständen, welche sich bei der Bewegung des Haares ablösen und den Eindruck vermitteln, der Anwender des entsprechenden Stylingmittels hätte Schuppen. Die mit den im Stand der Technik bekannten synthetischen Polymeren und Polymerkombinationen erhältlichen Polymerfilme weisen zwar einen zufriedenstellenden Halt auf, jedoch lässt dieser erzielte Frisurenhalt bei feuchter Umgebung stark nach. Dieser verschlechterte Halt bei feuchter Umgebung entspricht jedoch nicht dem Verbraucherbedürfnis nach einem gleichbleibend hohen Langzeithalt unabhängig von den äußeren Witterungseinflüssen. Es besteht daher ein Bedürfnis, die Feuchtebeständigkeit von durch Polymere und Polymerkombinationen erhältlichen Polymerfilme zu verbessern, ohne jedoch die anderen zuvor angeführten Eigenschaften von kosmetischen Mitteln, insbesondere Stylingmitteln, negativ zu beeinflussen

Aufgabe der vorliegenden Erfindung war es daher, neue Polymerkombinationen bereit zu stellen, welche neben einem hohen Langzeithalt, einer hohen Flexibilität und einer geringen Klebrigkeit auch eine hervorragende Feuchtebeständigkeit - insbesondere Schweiß- und Wasserbeständigkeit - aufweisen. Weiterhin sollen diese Polymerkombinationen die Formulierung stabil viskoser sowie transparenter kosmetischer Mittel zur temporären Verformung keratinischer Fasern ermöglichen.

Es wurde nun überraschend gefunden, dass eine Kombination eines Copolymers A auf Basis von Polyvinylpyrrolidon mit einem Copolymer B auf Basis von Acrylaten zu Stylingmitteln führt, welche einen hohen Halt und eine gute Flexibilität bei gleichzeitig hervorragender Feuchtebeständigkeit aufweisen. Überraschenderweise führt die Kombination der zuvor angeführten Polymere zu einem synergistischen Effekt in Bezug auf die Feuchtebeständigkeit, welche mit Hilfe des HHCR-Tests (High Humidity Curl Retention Test) ermittelt wurde. Die für eine angenehme Produkthaptik erforderliche geringe Klebrigkeit der kosmetischen Mittel wurde durch die erfindungsgemäße Polymerkombination nicht nachteilig beeinflusst. Weiterhin können bei Einsatz dieser Polymerkombination stabil viskose und transparente kosmetische Mittel formuliert werden.

Ein erster Gegenstand der vorliegenden Erfindung ist somit ein kosmetisches Mittel zur temporären Verformung keratinischer Fasern, enthaltend in einem kosmetisch verträglichen Träger

a) mindestens ein Copolymer A, bestehend aus den Struktureinheiten der Formel (I), (IIa) und (IIIa)

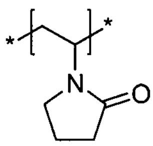 (I) 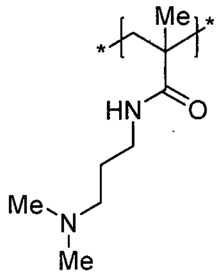 (IIa) 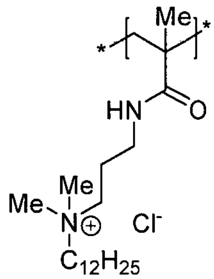 (IIIa),,

b) mindestens ein Copolymer B, bestehend aus mindestens einer Struktureinheit der Formel (IVa) und/oder mindestens einer Struktureinheit der Formel (IVb) und mindestens einer Struktureinheit der Formel (Va) und/oder mindestens einer Struktureinheit der Formel (Vb) und mindestens einer Struktureinheit der Formel (VIa)

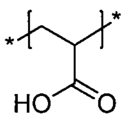 (IVa) 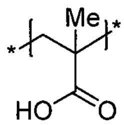 (IVb) 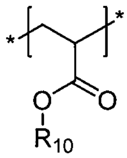 (Va) 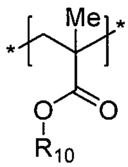 (Vb) 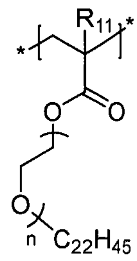 (VIa)

worin

$R_{10}$ für eine $C_{1}$-$_{6}$-Alkylgruppe steht,
$R_{11}$ für Wasserstoff oder eine Methylgruppe steht, und
n für ganze Zahlen von 22 bis 26 steht, und

c) mindestens eine alkalische Verbindung,
wobei das mindestens eine Copolymer A in einer Gesamtmenge von 0,01 bis 5,0 Gew.-%, bezogen auf das Gesamtgewicht des kosmetischen Mittels, enthalten ist,
wobei das mindestens eine Copolymer B in einer Gesamtmenge von 0,01 bis 5,0 Gew.-%, bezogen auf das Gesamtgewicht des kosmetischen Mittels, enthalten ist, und
wobei das kosmetische Mittel ein Gewichtsverhältnis der Gesamtmenge des mindestens einen Copolymers A zu der Gesamtmenge des mindestens einen Copolymers B von 2 : 1 bis 1 : 2 aufweist.

Durch die Kombination eines Copolymers A auf Basis von Polyvinylpyrrolidon mit einem Copolymer B auf Basis von Acrylaten wird ein synergistischer Effekt in Bezug auf die verbesserte Feuchtebeständigkeit der kosmetischen Mittel erreicht. Die erfindungsgemäßen Mittel gewährleisten daher selbst bei wechselnden äußerlichen Umwelteinflüssen einen dauerhaften und gleichbleibend hohen Frisurenhalt. Weiterhin führt der Einsatz der zuvor angeführten Polymerkombination nicht zu einem negativen Einfluss auf die weiteren Produkteigenschaften, wie beispielsweise die Klebrigkeit der Produkte. Zudem weist diese Polymerkombination auch keine negativen Auswirkungen auf die Viskosität sowie die gewünschte Transparenz der kosmetischen Mittel auf.

Gemäß obiger Formeln und aller folgenden Formeln steht eine chemische Bindung, welche mit dem Symbol "*" gekennzeichnet ist, für eine freie Valenz des entsprechenden Strukturfragments. Unter freier Valenz ist hierbei die Anzahl von Atombindungen zu verstehen, welche von dem entsprechenden Strukturfragment an der mit dem Symbol "*" gekennzeichneten Position ausgehen. Im Rahmen der vorliegenden Erfindung geht bevorzugt jeweils eine Atombindung von den mit dem Symbol "*" gekennzeichneten Positionen der Strukturfragmente zu weiteren Strukturfragmenten aus.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Im Rahmen der vorliegenden Erfindung werden unter dem Begriff "alkalische Verbindungen" solche Verbindungen verstanden, welche in protischen Lösungsmitteln Hydroxidionen abspalten können bzw. welche mit Säuren durch Neutralisation Salze bilden können.

Zudem sind unter dem Begriff der "Fettsäure", wie er im Rahmen der vorliegenden Erfindung verwendet wird, aliphatische Carbonsäuren zu verstehen, welche unverzweigte oder verzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Schließlich sind unter dem Begriff des "Fettalkohols" im Rahmen der vorliegenden Erfindung aliphatische, einwertige, primäre Alkohole zu verstehen, welche unverzweigte oder verzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Als ersten wesentlichen Bestandteil a) enthält das erfindungsgemäße kosmetische Mittel mindestens ein Copolymer A, welches mindestens aus den Struktureinheiten der Formel (I) und (IIa) und (IIIb) besteht.

Besonders gute Ergebnisse im Hinblick auf die Feuchtebeständigkeit der erfindungsgemäßen kosmetischen Mittel und somit den Halt unter verschiedenen äußeren Witterungseinflüssen werden erhalten, wenn das Copolymer A aus den Struktureinheiten der Formel (I), (IIa) und (IIIa)

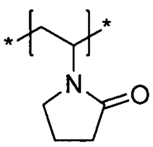 (I) 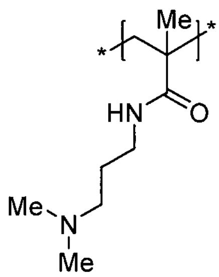 (IIa) 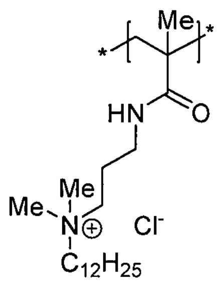 (IIIa),

besteht. Der Einsatz derartiger Copolymere A in Kombination mit dem mindestens einen speziellen Copolymer B führt zu einem synergistischen Effekt in Bezug auf die Feuchtebeständigkeit im Vergleich zu der Feuchtebeständigkeit, welche für die einzelnen Copolymere erhalten wurde.

Ein im Rahmen dieser Ausführungsform ganz besonders bevorzugtes Copolymer A ist ein Polymer mit der INCI-Bezeichnung Polyquaternium-55 (CAS Nummer 306769-73-3). Dieses Polymer ist ein Terpolymer aus Vinylpyrrolidon, Dimethylaminopropylmethacrylamid und (Meth)acryloylaminopropyllauryldimethylammoniumchlorid. Solche Polymere werden beispielsweise unter dem Handelsnamen Styleze W20 von der Firma ISP vertrieben.

Die kosmetischen Mittel enthalten das mindestens eine Copolymer A in einer Gesamtmenge von 0,01 bis 5,0 Gew.-%, vorzugsweise von 0,05 bis 4,0 Gew.-%, bevorzugt von 0,1 bis 3,0 Gew.-%, insbesondere von 0,2 bis 1,5 Gew.-%,, bezogen auf das Gesamtgewicht des kosmetischen Mittels. Die Verwendung dieser Mengen des Copolymers A resultiert in dem zuvor angeführten synergistischen Effekt in Bezug auf die Feuchtebeständigkeit bei Kombination mit dem speziellen Copolymer B. Weiterhin gewährleisten diesen Mengen neben der hervorragenden Feuchtebeständigkeit auch einen exzellenten Haltegrad der erfindungsgemäßen kosmetischen Mittel. Zudem wird bei Einsatz dieser Mengen sowohl eine stabile Viskosität dieser Mittel als auch eine ausreichende Transparenz dieser Mittel gewährleistet.

Als zweiten wesentlichen Bestandteil b) enthält das erfindungsgemäße kosmetische Mittel mindestens ein Copolymer B, welches aus den Struktureinheiten der Formeln (IVa) und/oder (IVb) und (Va) und/oder (Vb) und (VIa) besteht. In den Struktureinheiten der Formeln (Va) und (Vb) können die Reste $R_{10}$ für $C_1$-$C_6$-Alkylgruppen stehen.

Beispiele für derartige Gruppen sind Methyl-, Ethyl-, Propyl-, Isopropyl-, Hydroxpropyl, Butyl-, Sec-Butyl-, Isobutyl-, tert-Butyl-, Hydroxybutyl-, Pentyl- und Hexylgruppen. Weiterhin kann der Rest $R_{12}$ in der Struktureinheit der Formel (VI) für eine $C_6$-$C_{30}$-Alkylgruppe stehen. Derartige Gruppen sind beispielsweise Capryl-, Caprin-, Lauryl-, Myristyl-, Cetyl-, Stearyl-, Arachidyl-, Behenyl-, Oleyl- und Cerylgruppen.

Das Copolymer B besteht aus den Struktureinheiten der Formeln (IVa) und/oder (IVb) und (Va) und/oder (Vb) und (VIa)

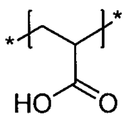 (IVa) 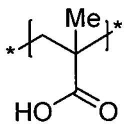 (IVb) 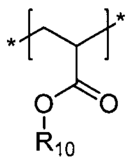 (Va) 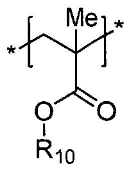 (Vb) 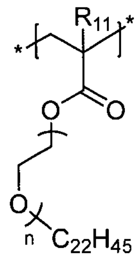 (VIa)

worin

$R_{10}$ für eine $C_1$-$_6$-Alkylgruppe steht,
$R_{11}$ für Wasserstoff oder eine Methylgruppe steht, und
n für ganze Zahlen von 22 bis 26 steht,

enthält. Die Verwendung derartiger Copolymere B führt in Verbindung mit dem Copolymer A zu einer signifikanten Erhöhung der Feuchtebeständigkeit.

Ein als Copolymer B besonders bevorzugt eingesetztes Polymer ist ein Polymer mit der INCI-Bezeichnung Acrylates/Beneneth-25 Methacrylate Copolymer. Dieses ist im Handel unter der Bezeichnung Aculyn 28 von der Firma Rohm&Haas erhältlich, hat einen Feststoffgehalt von etwa 19,0 bis 21,0 Gew.-% und einen pH-Wert von 3,5 bis 4,0.

Die kosmetischen Mittel enthalten das mindestens eine Copolymer B in einer Gesamtmenge von 0,01 bis 5,0 Gew.-%, vorzugsweise von 0,05 bis 4,0 Gew.-%, bevorzugt von 0,1 bis 3,0 Gew.-%, insbesondere von 0,2 bis 1,5 Gew.-%, bezogen auf das Gesamtgewicht des kosmetischen Mittels. Die Verwendung der zuvor genannten Mengen des Copolymers B führt in Kombination mit dem Copolymer A zu einem synergistischen Effekt in Bezug auf die Feuchtebeständigkeit der kosmetischen Mittel. Weiterhin gewährleisten diese Mengen einen hohen Halt der kosmetischen Mittel und eine ausreichende Flexibilität. Zudem treten bei Einsatz der zuvor angeführten Mengen keine unerwünschten Effekte auf die Klebrigkeit, die Viskosität und die Transparenz der erfindungsgemäßen kosmetischen Mittel auf.

Das kosmetische Mittel weist ein Gewichtsverhältnis der Gesamtmenge des mindestens einen Copolymers A zu der Gesamtmenge des mindestens einen Copolymers B von 2 : 1 bis 1 : 2 auf.

Die Copolymere A und/oder B werden in den erfindungsgemäßen kosmetischen Mitteln in teilneutraliserter oder neutralisierter Form eingesetzt. Zur Neutralisation enthalten diese Mittel daher mindestens eine alkalische Verbindung c).

Als vorteilhaft im Rahmen der vorliegenden Erfindung hat sich die Verwendung von als Alkanolaminen, insbesondere von primären Aminen mit einem $C_2$-$C_6$-Alkylgrundkörper, der mindestens eine Hydroxylgruppe trägt, erwiesen. Bevorzugt ist daher die alkalische Verbindung c) ausgewählt ist aus der Gruppe von 2-Aminoethan-1-ol (Monoethanolamin), Tris(2-hydroxyethyl)-amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan- 3-ol, 1-Aminopentan-4-ol, 2-Amino-2-methylpropan-1-ol, 1-Amino-2-methylpropan-2-ol, 3- Aminopropan-1,2-diol, 2-Amino-2-methylpropan-1,3-diol, insbesondere 2-Amino-2-methylpropan-1-ol. Erfindungsgemäß besonders bevorzugte kosmetische Mittel enthalten daher 2-Amino-2-methylpropanol als alkalische Verbindung c).

Die alkalische Verbindung c) wird vorzugsweise in einer Menge eingesetzt, welche die zur Neutralisation der Copolymere A und/oder B benötigte Menge nicht überschreitet. Vorzugsweise beträgt die in den erfindungsgemäßen Zusammensetzungen eingesetzte Menge an alkalischer Verbindung c) 80 bis 100%, besonders bevorzugt 90 bis 100% und insbesondere 95 bis 100% der zur vollständigen Neutralisation der Copolymere A und/oder B benötigten Menge. In einer bevorzugten Ausführungsform ist die alkalische Verbindung c) daher in einer Gesamtmenge von 0,05 bis 7,0 Gew.-%, vorzugsweise von 0,1 bis 5,0 Gew.-%, bevorzugt von 0,1 bis 4,0 Gew.-%, insbesondere von 0,1 bis 3,0 Gew.-%, bezogen auf das Gesamtgewicht des kosmetischen Mittels, enthalten.

Die erfindungsgemäßen kosmetischen Mittel enthalten die Copolymere A und B, die alkalische Verbindung c) sowie gegebenenfalls weitere Inhaltsstoffe in einem kosmetisch verträglichen Träger.

Bevorzugte kosmetisch verträgliche Träger sind wässrige, alkoholische oder wässrig-alkoholische Medien mit vorzugsweise mindestens 10 Gew.-% Wasser, bezogen auf das Gesamtgewicht des kosmetischen Mittels.

Besonders bevorzugt enthält der kosmetisch verträgliche Träger Wasser, insbesondere in einer Menge, dass das kosmetische Mittel, berechnet auf das Gesamtgewicht des kosmetischen Mittels, mindestens 10 Gew.-%, vorzugsweise mindestens 20 Gew.-%, insbesondere mindestens 40 Gew.-% Wasser, enthält. Ganz besonders bevorzugte kosmetische Mittel weisen, bezogen auf ihre Gesamtgewicht, einen Wasseranteil von 50 bis 95 Gew.-%, vorzugsweise von 60 bis 90 Gew.-%, insbesondere von 65 bis 85 Gew.-% auf.

Als Alkohole können insbesondere die für kosmetische Zwecke üblicherweise verwendeten niederen Alkohole

mit 1 bis 4 Kohlenstoffatomen, beispielsweise Ethanol und Isopropanol, enthalten sein.

Beispiele für wasserlösliche Lösungsmittel als Cosolvens sind Glycerin und/oder Ethylenglykol und/oder 1,2-Propylenglykol, welche in einer Menge von 0 bis 30 Gew.-%, bezogen auf das Gesamtgewicht des kosmetischen Mittels, eingesetzt werden können.

Der Langzeithalt des erfindungsgemäßen kosmetischen Mittels kann weitergehend gesteigert werden, wenn dieses mindestens ein weiteres filmbildendes und/oder festigendes Polymer enthält, welches von dem Copolymer A und dem Copolymer B verschieden ist. Es kann jedoch auch bevorzugt sein, als filmbildende und/oder festigende Polymere ausschließlich das zuvor angeführte Copolymer A und das zuvor angeführte Copolymer B einzusetzen.

Filmbildende bzw. festigende Polymere tragen durch Filmbildung zum Halt der aufgeprägten Form des Faserkollektivs, z.B. der Gesamtfrisur, bei. Die Filmbildung kann dabei durchaus punktuell sein und nur einige Fasern miteinander verbinden. Unter filmbildenden bzw. festigenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, welche eine ausreichende Löslichkeit in Wasser oder Wasser/Alkohol-Gemischen besitzen, um in den erfindungsgemäßen kosmetischen Mitteln in vollständig gelöster Form vorzuliegen. Die filmbildenden Polymere können synthetischen oder natürlichen Ursprungs sein. Unter filmbildenden Polymeren werden weiterhin auch solche Polymere verstanden, welche bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

In diesem Zusammenhang kann es erfindungsgemäß insbesondere vorgesehen sein, dass das weitere filmbildende und/oder festigende Polymer ausgewählt ist aus Copolymeren von Polyvinylpyrrolidon mit Vinylacetat. Einsetzbar im Rahmen der vorliegenden Erfindung sind insbesondere Polyvinylpyrrolidon/Vinylacetat-Copolymere mit einem molaren Verhältnis von Polyvinylpyrrolidon zu Vinylacetat von 70 zu 30, 60 zu 40, 50 zu 50 oder 30 zu 70. Derartige PVP/VA-Copolymere mit einem molaren Verhältnis von Polyvinylpyrrolidon zu Vinylacetat von 70 zu 30 sind beispielsweise unter den Handelsnamen PVP/VA E-735, PVP/VA I-735 und PVP/VA W-735 als 50%ige Dispersion in Ethanol, Isopropanol bzw. Wasser von der Firma Ashland erhältlich. PVP/VA-Copolymere mit einem molaren Verhältnis von Polyvinylpyrrolidon zu Vinylacetat von 60 zu 40 werden beispielsweise unter den Handelsnamen PVP/VA E-635 und PVP/VA W-635 als 50%ige Dispersion in Ethanol, Isopropanol bzw. Wasser von der Firma Ashland vertrieben. Unter den Handelsnamen PVP/VA E-535 und PVP/VA I-535 sind PVP/VA-Copolymere mit einem molaren Verhältnis von Polyvinylpyrrolidon zu Vinylacetat von 50 zu 50 als 50%ige Dispersion in Ethanol oder Isopropanol von der Firma Ashland erhältlich. Ein PVP/VA-Copolymer mit einem molaren Verhältnis von Polyvinylpyrrolidon zu Vinylacetat von 30 zu 70 wird beispielsweise unter dem Handelsnamen PVP/VA I-335 als 50%ige Dispersion in Isopropanol von der Firma Ashland vertrieben.

Neben den Copolymeren von Polyvinylpyrrolidon mit Vinylacetat können die erfindungsgemäßen kosmetischen Mittel in diesem Zusammenhang weitere filmbildende und/oder festigende Polymere enthalten, welche ausgewählt sind aus der Gruppe von Polymeren von Vinylpyrrolidon, Copolymeren von Vinylpyrrolidon mit Vinylimidazol und/oder Acrylamid und/der (Meth)acrylamid, Copolymeren des Isobutens, sowie deren Mischungen. Auch diese Polymere sind von dem Copolymer A und dem vernetzten Polyurethan-Vinyl-Polymer B verschieden.

Diese filmbildenden und/oder festigenden Polymere werden wiederum bevorzugt ausgewählt aus mindestens einem Polymer der Gruppe, welche gebildet wird aus

- Polyvinylpyrrolidon,
- Copolymeren aus N-Vinylpyrrolidon und N-Vinylimidazol und (Meth)acrylamid,
- Copolymeren aus N-Vinylpyrrolidon und N-Vinylimidazol und Acrylamid,
- Copolymeren aus N-Vinylpyrrolidon mit N,N-Di-$C_1$-$C_4$-Alkylamino-$C_2$-$C_4$-alkylacrylamid,
- Copolymeren aus N-Vinylpyrrolidon mit N,N-Di-$C_1$-$C_4$)-Alkylamino-$C_2$-$C_4$-alkylacrylamid,
- Copolymere des Isobutens.

Geeignete Polyvinylpyrrolidone sind beispielsweise Handelsprodukte wie Luviskol® K 90 oder Luviskol® K 85 der Firma BASF SE. Geeignete Polyvinylalkohole werden beispielsweise unter den Handelsbezeichnungen Elvanol® von Du Pont oder Vinol® 523/540 von der Firma Air Products vertrieben.

Die weiteren filmbildenden und/oder festigenden Polymere können in den erfindungsgemäßen kosmetischen Mitteln bevorzugt in einer Gesamtmenge von 0,1 Gew.-% bis 12,0 Gew.-%, vorzugsweise von 0,2 Gew.-% bis 10,0 Gew.-%, insbesondere von 0,5 Gew.-% bis 8,0 Gew.-%, jeweils bezogen auf das Gesamtgewicht des kosmetischen Mittels, enthalten sein.

Gemäß einer besonders bevorzugten Ausführungsform der vorliegenden Erfindung liegt das kosmetische Mittel als Gel vor. Unter dem Begriff "Gel" wird im Rahmen der vorliegenden Erfindung ein formbeständiges und leicht deformierbares System aus zwei Komponenten verstanden, wobei die eine Komponente in Form des Verdickungsmittels

bzw. Gelbildners ein räumliches, dreidimensionales Netzwerk ausbildet in dessen Räumen bzw. Poren die zweite Komponente in Form von Flüssigkeit, insbesondere Wasser, eingelagert ist.

Um die gelartige Konsistenz zu erreichen, werden vorteilhafter Weise weitere, als Verdickungsmittel oder Gelbildner wirkende Komponente(n), die von dem Copolymer A und dem vernetzten Polyurethan-Vinyl-Copolymer B verschieden ist/sind, eingesetzt. Die Gesamtmenge dieser Verdickungsmittel am Gesamtgewicht der kosmetischen Mittel beträgt beispielsweise 0,02 bis 3 Gew.-%, vorzugsweise 0,05 bis 1,5 Gew.-%, insbesondere 0,2 bis 0,8 Gew.-%.

Beispiele sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates/$C_{1-2}$ Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/$C_{1-18}$ Alkyl Acrylates/$C_{1-8}$ Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis- Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/Vinyl Caprolactam/DMAPA Acrylates Copolymer, Yeast Palmitate und Styrene/VP Copolymer.

Das Verdickungsmittel oder der Gelbildner ist bevorzugt in einer Gesamtmenge von 0,02 bis 3 Gew.-%, vorzugsweise von 0,05 bis 1,5 Gew.-%, insbesondere von 0,2 bis 0,8 Gew.-%, bezogen auf das Gesamtgewicht des kosmetischen Mittels, enthalten.

Ein im Rahmen der vorliegenden Erfindung besonders bevorzugt eingesetztes Verdickungsmittel ist eine Homopolyacrylsäure (INCI: Carbomer), welche im Handel unter dem Namen Carbopol® in unterschiedlichen Ausführungen erhältlich ist.

Nachfolgend ist eine besonders bevorzugte Ausführungsform der erfindungsgemäßen kosmetischen Mittel

aufgeführt:

Kosmetisches Mittel zur temporären Verformung keratinischer Fasern, enthaltend in einem kosmetisch verträglichen Träger - bezogen auf sein Gesamtgewicht -

a) 1,0 bis 5,0 Gew.-% mindestens eines Copolymers A, bestehend aus Struktureinheiten der Formeln (I) und (IIa) und (IIIa),

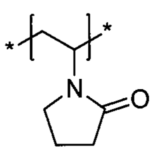 (I) 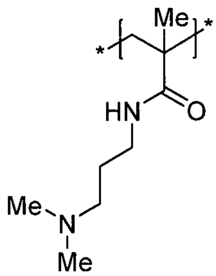 (IIa) 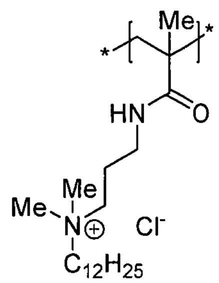 (IIIa),

b) 1,2 bis 3,2 Gew.-% mindestens eines Copolymers B, bestehend aus Struktureinheiten der Formeln (IVa) und/oder (IVb) und (Va) und/oder (Vb) und (VIa)

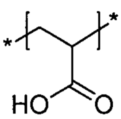 (IVa) 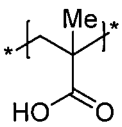 (IVb) 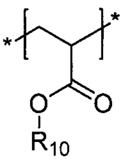 (Va) 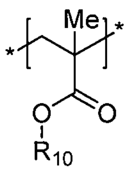 (Vb) 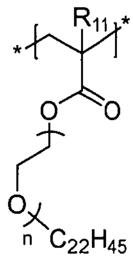 (VIa)

21

worin

- $R_{10}$ für eine $C_{1-6}$-Alkylgruppe steht,
- $R_{11}$ für Wasserstoff oder eine Methylgruppe steht, und
- n für ganze Zahlen von 22 bis 26 steht,

c) 0,05 bis 7,0 Gew.-%, vorzugsweise 0,1 bis 5,0 Gew.-%, bevorzugt 0,1 bis 4,0 Gew.-%, insbesondere 0,1 bis 3,0 Gew.-% mindestens einer alkalischen Verbindung c) in Form von 2-Amino-2-methylpropan-1-ol,

d) 0 bis 10 Gew.-%, vorzugsweise 2,0 bis 8,5 Gew.-%, insbesondere 3,0 bis 7,0 Gew.-%, mindestens eines Vinylpyrrolidonpolymers und/oder Vinylpyrrolidon/Vinylacetat-Copolymers, und

e) 0 bis 3 Gew.-%, vorzugsweise 0,05 bis 1,5 Gew.-%, insbesondere 0,2 bis 0,8 Gew.-%, einer Homopolyacrylsäure.

Die zuvor genannte besonders bevorzugte Ausführungsform der erfindungsgemäßen kosmetischen Mittel zeichnet sich durch eine hervorragende Feuchtebeständigkeit sowie durch einen guten Langzeithalt aus. Weiterhin weisen die aus den genannten Polymerkombinationen gebildeten Polymerfilme eine ausreichende Flexibilität auf, so dass die Bildung von Filmplaken vermieden wird. Zudem zeichnet sich dieses Mittel durch eine stabile Viskosität sowie eine hohe Transparenz aus.

Neben den zuvor beschriebenen Komponenten können die erfindungsgemäßen kosmetischen Mittel weitere Inhaltsstoffe enthalten. Zur Gruppe dieser weiteren Inhaltsstoffe zählen insbesondere die kosmetisch wirksamen Hilfs- und Zusatzstoffe, insbesondere zusätzliche Pflegestoffe.

Als Pflegestoff kann beispielsweise ein Silikonöl und/oder ein Silikongum eingesetzt werden. Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane, insbesondere PEG-12 Dimethicone und PEG-14 Dimethicone.

Als Pflegestoff einer anderen Verbindungsklasse kann das erfindungsgemäße kosmetische Mittel beispielsweise mindestens ein Proteinhydrolysat und/oder eines seiner Derivate enthalten. Proteinhydrolysate sind Produktgemische, welche durch sauer, basisch oder enzymatisch katalysierten Abbau von Proteinen (Eiweißen) erhalten werden. Unter dem Begriff "Proteinhydrolysate" werden erfindungsgemäß auch Totalhydrolysate aus einzelnen Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000 Dalton, vorzugsweise beträgt das Molgewicht 75 bis 50.000 Dalton, insbesondere 75 bis 20.000 Dalton.

Als Pflegestoff kann das erfindungsgemäße kosmetische Mittel weiterhin mindestens ein Vitamin, ein Provitamin, eine Vitaminvorstufe und/oder deren Derivate enthalten. Dabei sind erfindungsgemäß solche Vitamine, Provitamine und Vitaminvorstufen bevorzugt, welche üblicherweise den Gruppen A, B, C, E, F und H zugeordnet werden.

Der Zusatz von Panthenol kann die Flexibilität des bei Anwendung des erfindungsgemäßen kosmetischen Mittels gebildeten Polymerfilms.

Als Pflegestoff können die erfindungsgemäßen kosmetischen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen zu zählen.

Esteröle, also Ester von $C_6$ - $C_{30}$ - Fettsäuren mit $C_2$ - $C_{30}$ - Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

Weiterhin sind in den erfindungsgemäßen kosmetischen Mitteln bevorzugt Emulgatoren bzw. oberflächenaktive Mittel enthalten. Bevorzugt sind PEG-Derivate von hydriertem Ricinusöl, welche beispielsweise unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil oder PEG-40 Hydrogenated Castor Oil. Erfindungsgemäß bevorzugt ist die Verwendung von PEG-40 Hydrogenated Castor Oil. Diese sind bevorzugt in einer Gesamtmenge von 0,05 bis 1,5 Gew.-%, vorzugsweise von 0,1 bis 1,0 Gew.-%, bevorzugt von 0,2 bis 0,8 Gew.-%, insbesondere von 0,3 bis 0,6 Gew.-%, bezogen auf das Gesamtgewicht des kosmetischen Mittels, enthalten.

Die kosmetischen Mittel der vorliegenden Erfindung können in den für die temporäre Umformung von keratinischen Fasern, insbesondere Haaren, üblichen Formen konfektioniert sein, beispielsweise als Haargel, Haarspray, Haarschaum oder Haarwachs. Bevorzugt ist die Konfektionierung als Haargel.

Sowohl Haarschäume als auch Haarsprays erfordern die Anwesenheit von Treibmitteln und sind als sogenannte Aerosole konfektioniert. Als druckfeste Behälter für derartige Aerosole kommen Gefäße aus Metall (Aluminium, Weißblech, Zinn), geschütztem bzw. nicht-splitterndem Kunststoff oder aus Glas, welcher außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber dem im Druckbehälter befindlichen erfindungsgemäßen kosmetischen Mittel. Besonders bevorzugt weisen die verwendeten Ventile einen innenlackierten Ventilteller auf, wobei Lackierung und Ventilmaterial miteinander kompatibel sind. Werden Aluminiumventile eingesetzt, so können deren Ventilteller innen z. B. mit Micoflex-Lack beschichtet sein. Werden erfindungsgemäß Weißblechventile eingesetzt, so können deren Ventilteller innen z. B. mit PET (Polyethylenterephthalat) beschichtet sein. Über das Mengenverhältnis von Treibmittel zu den übrigen Bestandteilen der kosmetischen Mittel lassen sich bei gegebener Sprühvorrichtung die Größen der Aerosoltröpfchen und die jeweilige Größenverteilung einstellen.

Wenn das erfindungsgemäße kosmetische Mittel ein Treibmittel enthält, ist dieses vorteilhafterweise in einer Gesamtmenge von 10 bis 80 Gew.-%, vorzugsweise von 20 bis 70 Gew.-%, insbesondere von 30 bis 60 Gew.-%, bezogen auf das Gesamtgewicht des kosmetischen Mittels, enthalten.

Erfindungsgemäß geeignete Treibmittel sind beispielsweise ausgewählt aus $N_2O$, Dimethylether, $CO_2$, Luft, Alkanen mit 3 bis 5 Kohlenstoffatomen, wie Propan, n-Butan, iso-Butan, n-Pentan und iso-Pentan, sowie deren Mischungen. Bevorzugt sind Dimethylether, Propan, n-Butan, iso-Butan und Mischungen daraus. Gemäß einer bevorzugten Ausführungsform werden die genannten Alkane, Mischungen der genannten Alkane oder Mischungen der genannten Alkane mit Dimethylether als einziges Treibmittel eingesetzt. Die vorliegende Erfindung umfasst aber ausdrücklich auch die Mitverwendung von Treibmitteln vom Typ der Fluorchlorkohlenwasserstoffe, insbesondere aber der Fluorkohlenwasserstoffe.

Ganz besonders bevorzugt werden Dimethylether oder Mischungen von Propan und Butan als alleiniges Treibmittel im Gewichtsverhältnis Propan zu Butan von 20 zu 80 bis 15 zu 85 verwendet. Die Mischungen werden wiederum bevorzugt in den erfindungsgemäßen Mitteln in einer Gesamtmenge von 30 bis 55 Gew.-% - bezogen auf das Gesamtgewicht des kosmetischen Mittels - eingesetzt. Unter Butan wird erfindungsgemäß n-Butan, iso-Butan und Gemische aus n-Butan und iso-Butan verstanden.

Ein weiterer Gegenstand der vorliegenden Erfindung ist ein Verfahren zur temporären Verformung keratinischer Fasern, wobei ein erfindungsgemäßes kosmetisches Mittel auf die keratinischen Fasern appliziert und diese anschließend in die gewünschte Form gebracht werden.

Bezüglich weiterer bevorzugter Ausführungsformen des erfindungsgemäßen Verfahrens gilt mutatis mutandis das zu den erfindungsgemäßen kosmetischen Mitteln Gesagte.

Zudem ist ein weiterer Gegenstand der vorliegenden Erfindung die Verwendung eines erfindungsgemäßen kosmetischen Mittels zur temporären Verformung keratinischer Fasern.

Schließlich ist ein weiterer Gegenstand der vorliegenden Erfindung die Verwendung eines erfindungsgemäßen kosmetischen Mittels zur Verbesserung der Feuchtebeständigkeit temporär verformter keratinischer Fasern.

Bezüglich weiterer bevorzugter Ausführungsformen der erfindungsgemäßen Verwendungen gilt mutatis mutandis das zu den erfindungsgemäßen kosmetischen Mitteln und zu dem erfindungsgemäßen Verfahren Gesagte. Die nachfolgenden Beispiele erläutern die vorliegende Erfindung, ohne sie jedoch darauf einzuschränken:

Beispiele:

1. Formulierungen (alle Angaben in Gewichtsprozent, bezogen auf das Gesamtgewicht des jeweiligen kosmetischen Mittels):

| Rohstoff | V1 | V2 | E1 |
|---|---|---|---|
| Stvleze W 20 (Copolymer A) [1)] | 8,4 | -- | 4,2 |
| Aculvn 28 (Copolymer B) [2)] | -- | 8,4 | 4,2 |
| AMP-Ultra PC 2000 (alkalische Verbindung) [3)] | 0,18 | 0,12 | 0,30 |
| Wasser | ad 100 | ad 100 | ad 100 |
| [1)] INCI-Bezeichnung: Polyquaternium-55 (19-21 Gew.-%ige Dispersion, ISP)<br>[2)] INCI-Bezeichnung: Acrylates/Beheneth-25 Methacrylate Copolymer (19-21 Gew.-%ige Dispersion in Wasser, Rohm & Haas) | | | |

Die kosmetischen Mittel V1, V2 und E1 wurden durch Vermischen der obigen Inhaltsstoffe erhalten. Der Polymergehalt in den kosmetischen Mitteln V1, V2 und E1 beträgt jeweils 0,8 Gew.-%.

Die hergestellten kosmetischen Mittel wurden auf ihre Feuchtebeständigkeit mittels des HHCR-Tests (High Humidity Curl Retention-Tests) geprüft.

Hierzu wurden jeweils 10 Haarsträhnen (European natural, Typ 827560 an einer Seite abgebunden, nicht geklebt, Farbe 6/0, Länge 240 mm, Gewicht ~ 0,6 g, $L_{max}$ = 220 mm, Kerling Internationale Haarfarbrik GmbH) pro kosmetischem Mittel V1, V2 und E1 präpariert, indem 180 mg des jeweiligen kosmetischen Mittels auf die Haarsträhnen aufgetragen und mit der Hand einmassiert wurden. Anschließend wurden die Haarsträhnen auf Wickler (Länge 160 mm, Durchmesser 10 mm) aufgewickelt und über Nacht bei 298 K und 50 % relativer Luftfeuchtigkeit getrocknet. Nach Abwickeln der Haarsträhnen wurden diese an einem Metallgestell befestigt, in eine Klimakammer bei 298 K und 85 % relativer Luftfeuchtigkeit gestellt und deren Länge direkt im Anschluss mittels eines Lasers bestimmt (entspricht dem Wert $L_0$). Nach weiteren 6 h wurde erneut die Länge der Haarsträhnen bestimmt (entspricht Wert $L_t$).

Die Feuchtebeständigkeit, also der HHCR-Wert wird anhand der ermittelten Werte gemäß folgender Gleichung berechnet:

$$HHCR = \frac{L_{\max} - L_t}{L_{\max} - L_0} * 100$$

Die Ergebnisse wurden mittels des Mann Whitney U-Tests überprüft und waren signifikant.

In der nachfolgenden Tabelle sind die HHCR-Werte für die kosmetischen Mittel V1, V2 und E1 angegeben,

wobei der Erwartungswert E(V1+V2) dem Mittelwert der HHCR-Werte der beiden einzelnen Copolymere A und B entspricht.

| | V1 | V2 | E(V1+V2) | E1 |
|---|---|---|---|---|
| HHCR [%] | 64 | 73 | 69 | 80 |

Der für die erfindungsgemäße Kombination ermittelte HHCR-Wert (kosmetisches Mittel E1) liegt somit signifikant höher als der für diese Kombination zu erwartende HHCR-Wert (E(V1+V2)). Durch den Einsatz der erfindungsgemäßen Kombination eines Copolymers A auf Basis von Polyvinylpyrrolidon mit einem Copolymer B auf Basis von Acrylaten wird eine synergistische Steigerung der Feuchtebeständigkeit von kosmetischen Mitteln zur temporären Verformung keratinischer Fasern, insbesondere menschlicher Haare, erreicht.

## Patentansprüche

**1.** Kosmetisches Mittel zur temporären Verformung keratinischer Fasern, enthaltend in einem kosmetisch verträglichen Träger

a) mindestens ein Copolymer A, bestehend aus den Struktureinheiten der Formel (I), (IIa) und (Iila)

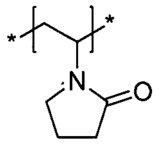 (I) 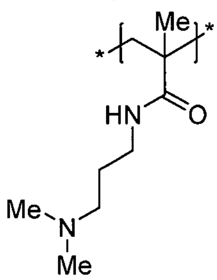 (IIa) 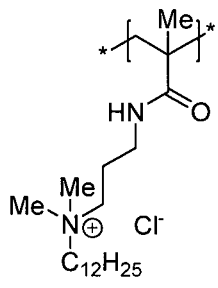 (IIIa),

b) mindestens ein Copolymer B, bestehend aus mindestens einer Struktureinheit der Formel (IVa) und/oder mindestens eine Struktureinheit der Formel (IVb) und mindestens eine Struktureinheit der Formel (Va) und/oder mindestens einer Struktureinheit der Formel (Vb) und mindestens einer Struktureinheit der Formel (VIa)

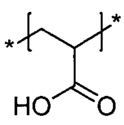 (IVa) 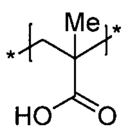 (IVb) 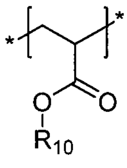 (Va) 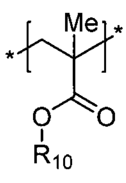 (Vb) 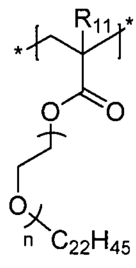 (VIa)

worin

$R_{10}$ für eine $C_1$-$_6$-Alkylgruppe steht,
$R_{11}$ für Wasserstoff oder eine Methylgruppe steht, und
n für ganze Zahlen von 22 bis 26 steht, und

c) mindestens eine alkalische Verbindung,
wobei das mindestens eine Copolymer A in einer Gesamtmenge von 0,01 bis 5,0 Gew.-%, bezogen auf das Gesamtgewicht des kosmetischen Mittels, enthalten ist,
wobei das mindestens eine Copolymer B in einer Gesamtmenge von 0,01 bis 5,0 Gew.-%, bezogen auf das Gesamtgewicht des kosmetischen Mittels, enthalten ist, und

wobei das kosmetische Mittel ein Gewichtsverhältnis der Gesamtmenge des mindestens einen Copolymers A zu der Gesamtmenge des mindestens einen Copolymers B von 2 : 1 bis 1 : 2 aufweist.

2. Kosmetisches Mittel nach Anspruch 1, **dadurch gekennzeichnet, dass** das mindestens eine Copolymer A in einer Gesamtmenge 0,05 bis 4,0 Gew.-%, bevorzugt von 0,1 bis 3,0 Gew.-%, insbesondere von 0,2 bis 1,5 Gew.-%, bezogen auf das Gesamtgewicht des kosmetischen Mittels, enthalten ist.

3. Kosmetisches Mittel nach einem der vorangehenden Ansprüche, **dadurch gekennzeichnet, dass** das mindestens eine Copolymer B in einer Gesamtmenge von 0,05 bis 4,0 Gew.-%, bevorzugt von 0,1 bis 3,0 Gew.-%, insbesondere von 0,2 bis 1,5 Gew.-%, bezogen auf das Gesamtgewicht des kosmetischen Mittels, enthalten ist.

4. Kosmetisches Mittel nach einem der vorangehenden Ansprüche, **dadurch gekennzeichnet, dass** die alkalische Verbindung c) ausgewählt ist aus der Gruppe von 2-Aminoethan-1-ol (Monoethanolamin), Tris(2-hydroxyethyl)-amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan- 3-ol, 1-Aminopentan-4-ol, 2-Amino-2-methylpropan-1-ol, 1-Amino-2-methylpropan-2-ol, 3- Aminopropan-1,2-diol, 2-Amino-2-methylpropan-1,3-diol, insbesondere 2-Amino-2-methylpropan-1-ol.

5. Kosmetisches Mittel nach einem der vorangehenden Ansprüche, **dadurch gekennzeichnet, dass** die alkalische Verbindung c) in einer Gesamtmenge von 0,05 bis 7,0 Gew.-%, vorzugsweise von 0,1 bis 5 Gew.-%, bevorzugt von 0,1 bis 4,0 Gew.-%, insbesondere von 0,1 bis 3,0 Gew.-%, bezogen auf das Gesamtgewicht des kosmetischen Mittels, enthalten ist.

6. Verfahren zur temporären Verformung keratinischer Fasern, wobei ein kosmetisches Mittel nach einem der Ansprüche 1 bis 5 auf die keratinischen Fasern appliziert und diese anschließend in die gewünschte Form gebracht werden.

7. Verwendung eines kosmetischen Mittels gemäß einem der Ansprüche 1 bis 5 zur temporären Verformung keratinischer Fasern.

## Claims

1. A cosmetic agent for temporarily shaping keratin fibers, containing, in a cosmetically acceptable carrier,

a) at least one copolymer A consisting of the structural units of formulae (I), (IIa) and (IIIa)

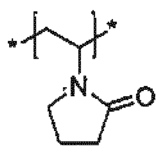 (I) 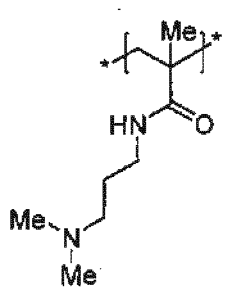 (IIa) 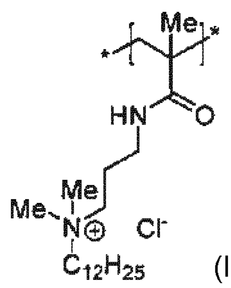 (IIIa).

b) at least one copolymer B consisting of at least one structural unit of formula (IVa) and/or at least one structural unit of formula (IVb) and at least one structural unit of formula (Va) and/or at least one structural unit of formula (Vb) and at least one structural unit of formula (Via)

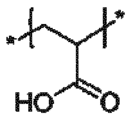 (IVa) 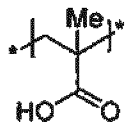 (IVb) 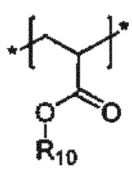 (Va) 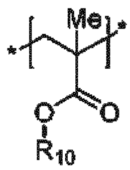 (Vb) 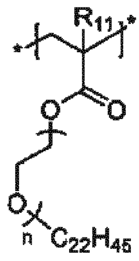 (VIa)

in which

$R_{10}$ represents a $C_{1-6}$ alkyl group,
$R_{11}$ represents hydrogen or a methyl group, and
n represents integers from 22 to 26, and

c) at least one alkaline compound, wherein the at least one copolymer A is contained in a total amount of from 0.01 to 5.0 wt.%, based on the total weight of the cosmetic agent, wherein the at least one copolymer B is contained in a total amount of from 0.01 to 5.0 wt.%, based on the total weight of the cosmetic agent, and wherein the cosmetic agent has a weight ratio of the total amount of the at least one copolymer A to the total amount of the at least one copolymer B of from 2:1 to 1:2.

2. The cosmetic agent according to claim 1, **characterized in that** the at least one copolymer A is contained in a total amount of from 0.05 to 4.0 wt.%, preferably from 0.1 to 3.0 wt.%, in particular from 0.2 to 1.5 wt.%, based on the total weight of the cosmetic agent.

3. The cosmetic agent according to one of the preceding claims, **characterized in that** the at least one copolymer B is contained in a total amount of from 0.05 to 4.0 wt.%, preferably from 0.1 to 3.0 wt.%, in particular from 0.2 to 1.5 wt.%, based on the total weight of the cosmetic agent.

4. The cosmetic agent according to one of the preceding claims, **characterized in that** the alkaline compound c) is selected from the group of 2-aminoethan-1-ol (monoethanolamine), tris(2-hydroxyethyl)amine (triethanolamine), 3-aminopropan-1-ol, 4-aminobutan-1-ol, 5-aminopentan-1-ol, 1-aminopropan-2-ol, 1-aminobutan-2-ol, 1-aminopentan-2-ol, 1-aminopentan-3-ol, 1-aminopentan-4-ol, 2-amino-2-methylpropan-1-ol, 1-amino-2-methylpropan-2-ol, 3-aminopropan-1,2-diol, 2-amino-2-methylpropan-1,3-diol, in particular 2-amino-2-methylpropan-1-ol.

5. The cosmetic agent according to one of the preceding claims, **characterized in that** the alkaline compound c) is contained in a total amount of from 0.05 to 7.0 wt.%, preferably from 0.1 to 5 wt.%, more preferably from 0.1 to 4.0 wt.%, in particular from 0.1 to 3.0 wt.%, based on the total weight of the cosmetic agent.

6. A method for temporarily shaping keratin fibers, wherein a cosmetic agent according to one of claims 1 to 5 is applied to the keratin fibers and these are subsequently shaped as desired.

7. The use of a cosmetic agent according to one of claims 1 to 5 for temporarily shaping keratin fibers.

**Revendications**

1. Composition cosmétique destinée à la mise en forme temporaire de fibres kératiniques, contenant, dans un support cosmétiquement acceptable,

a) au moins un copolymère A constitué des unités structurelles répondant à la formule (I), (IIa) et (IIIa)

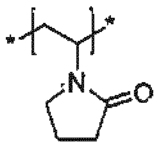 (I) 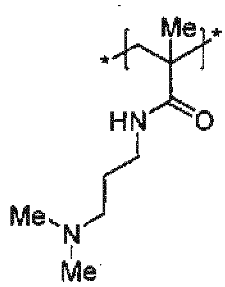 (IIa) 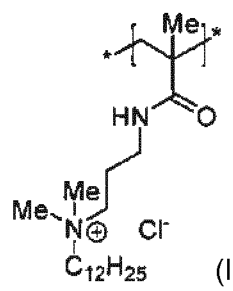 (IIIa),

b) au moins un copolymère B constitué d'au moins une unité structurelle répondant à la formule (IVa) et/ou au moins une unité structurelle répondant à la formule (IVb), et au moins une unité structurelle répondant à la formule (Va) et/ou au moins une unité structurelle répondant à la formule (Vb), et au moins une unité structurelle répondant à la formule (VIa)

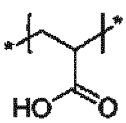 (IVa) 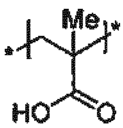 (IVb) 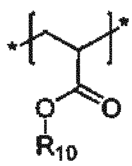 (Va) 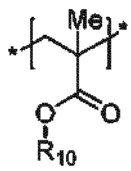 (Vb) 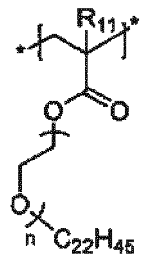 (VIa)

dans lesquelles

$R_{10}$ représente un groupe alkyle $C_{1-6}$,
$R_{11}$ représente un atome d'hydrogène ou un groupe méthyle, et
n représente des nombres entiers de 22 à 26, et

c) au moins un composé alcalin, l'au moins un copolymère A étant présent en une quantité totale de 0,01 à 5,0 % en poids par rapport au poids total de la composition cosmétique, l'au moins un copolymère B étant présent en une quantité totale de 0,01 à 5,0 % en poids par rapport au poids total de la composition cosmétique, et la composition cosmétique présentant un rapport en poids de la quantité totale de l'au moins un copolymère A par rapport à la quantité totale de l'au moins un copolymère B compris entre 2:1 et 1:2.

2. Composition cosmétique selon la revendication 1, **caractérisée en ce que** l'au moins un copolymère A est présent en une quantité totale de 0,05 à 4,0 % en poids, de préférence de 0,1 à 3,0 % en poids, en particulier de 0,2 à 1,5 % en poids par rapport au poids total de la composition cosmétique.

3. Composition cosmétique selon l'une des revendications précédentes, **caractérisée en ce que** l'au moins un copolymère B est présent en une quantité totale de 0,05 à 4,0 % en poids, de préférence de 0,1 à 3,0 % en poids, en particulier de 0,2 à 1,5 % en poids par rapport au poids total de la composition cosmétique.

4. Composition cosmétique selon l'une des revendications précédentes, **caractérisée en ce que** le composé alcalin c) est choisi dans le groupe constitué par le 2-aminoéthane-1-ol (monoéthanolamine), la tris(2-hydroxyéthyle)-amine (triéthanolamine), le 3-aminopropan-1-ol, le 4-aminobutane-1-ol, le 5-aminopentane-1-ol, le 1-aminopropane-2-ol, le 1-aminobutane-2-ol, le 1-aminopentane-2-ol, le 1-aminopentane-3-ol, le 1-aminopentane-4-ol, le 2-amino-2-méthylpropane-1-ol, le 1-amino-2-méthylpropane-2-ol, le 3-aminopropane-1,2-diol, le 2-amino-2-méthylpropane-1,3-diol, en particulier le 2-amino-2-méthylpropane-1-ol.

5. Composition cosmétique selon l'une des revendications précédentes, **caractérisée en ce que** le composé alcalin c) est présent en une quantité totale de 0,05 à 7,0 % en poids, de préférence de 0,1 à 5 % en poids, préférablement de 0,1 à 4,0 % en poids, en particulier de 0,1 à 3,0 % en poids par rapport au poids total de la composition cosmétique.

6. Procédé de mise en forme temporaire de fibres kératiniques, **caractérisé en ce qu'**une composition cosmétique

selon l'une des revendications 1 à 5 est appliquée sur les fibres kératiniques, et celles-ci sont ensuite mises sous la forme souhaitée.

7. Utilisation d'une composition cosmétique selon l'une des revendications 1 à 5, destinée à la mise en forme temporaire de fibres kératiniques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010020503 A
- WO 2013092062 A


### In der Beschreibung aufgeführte Nicht-Patentliteratur


- *CHEMICAL ABSTRACTS,* 306769-73-3